# EUROPEAN PATENT APPLICATION

(11) **EP 1 927 611 A1**
(43) Date of publication of application: **04.06.2008**
(21) Application number: 07022351.6
(22) Date of filing: 17.11.2007
(51) Int. Cl.: C08G 65/26, C07C 41/03, C07C 43/23

(54) **Continuous processes for the production of alkylphenol ethoxylates**

(30) Priority: 01.12.2006 US 607537
(71) Applicant: Bayer MaterialScience LLC, Pittsburgh, PA 15205 (US)
(72) Inventor: McDaniel, Kenneth, G., Charleston WV 25311 (US); Reese, Jack, R., II, Hurricane WV 25526 (US)
(74) Representative: Perchenek, Nils

(57) **Abstract**

The present invention provides continuous processes for the production of an alkylphenol ethoxylate from an alkylphenol in the presence of a double metal cyanide ("DMC") catalyst. The products made by the inventive continuous processes may offer advantages where the alkylphenol ethoxylate is used in or as a surfactant.

## Description

### FIELD OF THE INVENTION

The present invention relates in general to polymerization, and more specifically, to improved processes for the production of alkylphenol ethoxylates useful in or as surfactants and detergents.

### BACKGROUND OF THE INVENTION

Alkyl and alkylaryl ethoxylates are widely used in the detergents industry. Worldwide, the majority of ethoxylates used in detergents are produced via semi-batch processes utilizing base catalysis, typically potassium hydroxide ("KOH"). As such ethoxylates are commodity materials, the production economics and capability to manufacture the precursors are important determinants of profitability. Currently, only a small fraction of such surfactants are produced with specialized catalysts which give a narrow molecular weight distribution. Processes employing such "peaked catalysts" are reported to have higher manufacturing costs, likely related to higher catalyst costs and the need for catalyst removal following the ethoxylation process. Although the narrow distribution products from such processes can give higher performance in most applications, the cost driven focus of the detergent industry dictates the use of the KOH-catalyzed, wide distribution products because the benefits of enhanced performance are not sufficient to offset the increased costs.

The semi-batch process is the industry standard and although it is has been optimized and refined, there remain disadvantages to the use of this process. The semi-batch process operates with a headspace of ethylene oxide and an inerting gas and there are several nonproductive steps in the reactor sequence. Because pure ethylene oxide can present a hazard, the headspace must be inerted with nitrogen or low oxygen content gas and at the end of the cycle this inerting gas which may contain traces of ethylene oxide must be wasted. In the sequence of nonproductive steps, the alcohol alkylphenol is charged followed by catalyst and then the base is converted to the potassium or sodium phenoxide by stripping to remove water after the mixture is heated to process temperature. After oxide addition and digestion, the product is pumped from the reactor. Overall, the nonproductive steps can account for 50% of the reactor cycle time.

The preparation of phenolic-based polyethers is more problematic than those prepared from starters bearing aliphatic hydroxyl groups. The decreased basicity of alkali metal phenoxides and related compounds lowers the oxyalkylation rate considerably. In addition to slow oxyalkylation, phenolic starters exhibit a relatively long "induction time" prior to attaining a reasonable oxyalkylation rate.

Moreover, unlike the oxyalkylation of starters such as ethylene glycol, trimethylolpropane, and the like, where a homogenous or substantially homogenous reaction mixture is obtained during alkylene oxide addition, alkylene oxides are poorly soluble in phenolic compounds and vice versa.

Further, as taught by U.S. Pat. No. 6,624,333 issued to Koser, et al., alkali metal hydroxides, such as sodium hydroxide and potassium hydroxide, as a catalyst for alkoxylating phenolic compounds can lead to addition products having relatively broad molar mass distributions.

The use of double metal cyanide ("DMC") catalysts for the production of alkoxylates has been known since General Tire's development in the 1960's. In the 1970's, Herold in U.S. Pat. No. 3,829,505, described the preparation of high molecular weight diols, triols etc., using double metal cyanide catalysts. However, the catalyst activity, coupled with catalyst cost and the difficulty of removing catalyst residues from the polyol product, prevented commercialization of the products. There was limited utilization of the DMC technology until the 1990's when Le-Khac, in U.S. Pat. Nos. 5,470,813 and 5,482,908, demonstrated both improved catalysts and processes technologies that lowered the cost of production for polyols to be competitive with that for potassium hydroxide-based process for a wide range of polyols. However, even with these advancements, DMC catalyst technology has been applied mostly to the production of mixed oxide and all propylene oxide-based polyols.

Because of its unique features, the DMC catalyst provides a poor distribution of ethylene oxide when the equivalent weight of the initiator or base polyol is greater than about 800. The production of ethylene oxide ("EO") capped polyols suitable for use in polyurethanes requires either a two-stage system of DMC and potassium hydroxide or potassium hydroxide alone as the catalyst. Thus, although DMC catalysts are very effective for the production of ethoxylates when the starter equivalent weight is less than about 800, these capped low molecular weight polyols are not widely used for the production of polyurethanes.

The use of DMC as a catalyst for the production of semi-batch ethoxylates is disclosed in a number of patents and patent applications. For example, U.S. Pat. No. 6,821,308, issued Combs et al., teaches the alkoxylation of phenolic compounds with DMC. Although they demonstrate the use of propylene oxide as the alkylene oxide, Combs et al. do not teach or suggest the use of pure ethylene oxide.

Thus, there remains a need for improved surfactant production processes which would permit the use of alkylphenols and would not require a catalyst activation step to increase reactor productivity.

### SUMMARY OF THE INVENTION

Accordingly, the present invention provides a continuous process for the production of alkylphenol ethoxylates. The inventive continuous processes produce an alkylphenol ethoxylate from an alkylphenol in the presence of a double metal cyanide ("DMC") catalyst. The alkylphenol ethoxylates produced by the inventive continuous processes may provide advantages where the alkylphenol ethoxylate is used in or as a surfactant.

These and other advantages and benefits of the present invention will be apparent from the Detailed Description of the Invention herein below.

### BRIEF DESCRIPTION OF THE FIGURES

The present invention will now be described for purposes of illustration and not limitation in conjunction with the figures, wherein:
Figure 1 is a gel permeation chromatograph comparing the polydispersity of two mixed oxide triols;
Figure 2 is a gel permeation chromatograph comparing the polydispersity of two all propylene oxide diols;
Figure 3a is a diagram of a continuous stirred reactor ("CSTR") partitioned with a perforated plate;
Figure 3b is a top view of a perforated plate;
Figure 4a is a diagram of a continuous stirred reactor ("CSTR") partitioned with a rotation disk;
Figure 4b is a top view of a rotation disk; and
Figure 5 is a gel permeation chromatograph comparing the polydispersity of polyols produced by base catalysis and the inventive multi-stage DMC-catalyzed process.

### DETAILED DESCRIPTION OF THE INVENTION

The present invention will now be described for purposes of illustration and not limitation. Except in the operating examples, or where otherwise indicated, all numbers expressing quantities, percentages, OH numbers, functionalities and so forth in the specification are to be understood as being modified in all instances by the term "about." Equivalent weights and molecular weights given herein in Daltons (Da) are number average equivalent weights and number average molecular weights respectively, unless indicated otherwise.

The present invention provides a multi-stage continuous process for the production of an alkylphenol ethoxylate involving preparing a mixture of an alkylphenol and a double metal cyanide ("DMC") catalyst, establishing ethoxylation conditions in a first continuous stirred tank reactor ("CSTR"), continuously feeding ethylene oxide and the mixture of alkylphenol and DMC catalyst to the first CSTR reactor under conditions suitable to produce an ethoxylate, continuously feeding the reaction mixture from the first CSTR reactor and further ethylene oxide to a second CSTR reactor or to a tubular reactor under conditions suitable to produce an alkylphenol ethoxylate and continuously withdrawing the alkylphenol ethoxylate from the second CSTR reactor or tubular reactor to a collection vessel.

The present invention also provides a multi-stage continuous process for the production of an ethoxylate involving preparing a mixture of an alkylphenol and a double metal cyanide ("DMC") catalyst, establishing ethoxylation conditions in a first portion of a partitioned continuous stirred tank reactor ("CSTR"), continuously feeding ethylene oxide and the mixture of alkylphenol and DMC catalyst to the first portion of the CSTR reactor under conditions suitable to produce an ethoxylate, continuously forcing the reaction mixture from the first portion of the CSTR reactor into a second portion of the CSTR reactor and adding further ethylene oxide under conditions suitable to produce an alkylphenol ethoxylate and continuously withdrawing the alkylphenol ethoxylate from the second portion of the CSTR reactor to a collection vessel.

The present invention further provides a single stage continuous process for the production of an alkylphenol ethoxylate involving preparing a mixture of an alkylphenol and a double metal cyanide ("DMC") catalyst, establishing ethoxylation conditions in a reactor, continuously feeding ethylene oxide and the mixture of alkylphenol and DMC catalyst to the reactor under conditions suitable to produce an ethoxylate, continuously feeding further ethylene oxide to the reactor under conditions suitable to produce an alkylphenol ethoxylate and continuously withdrawing the alkylphenol ethoxylate from the reactor to a collection vessel.

The present invention yet further provides an improved process for the production of a surfactant, the improvement involving including the alkylphenol ethoxylate produced by either of the inventive multi-stage continuous processes or by the inventive single-stage process.

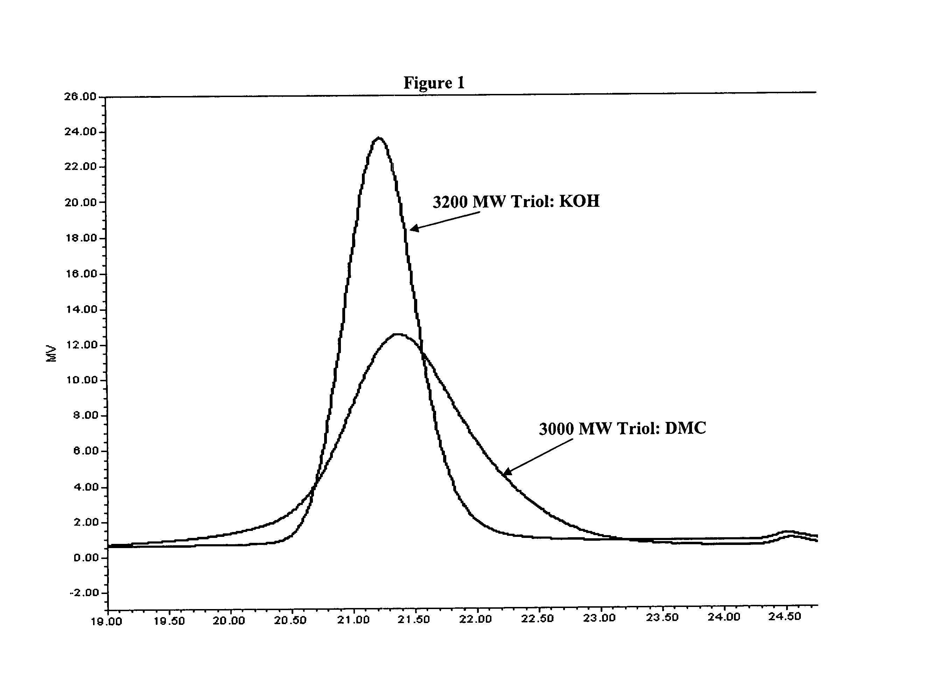

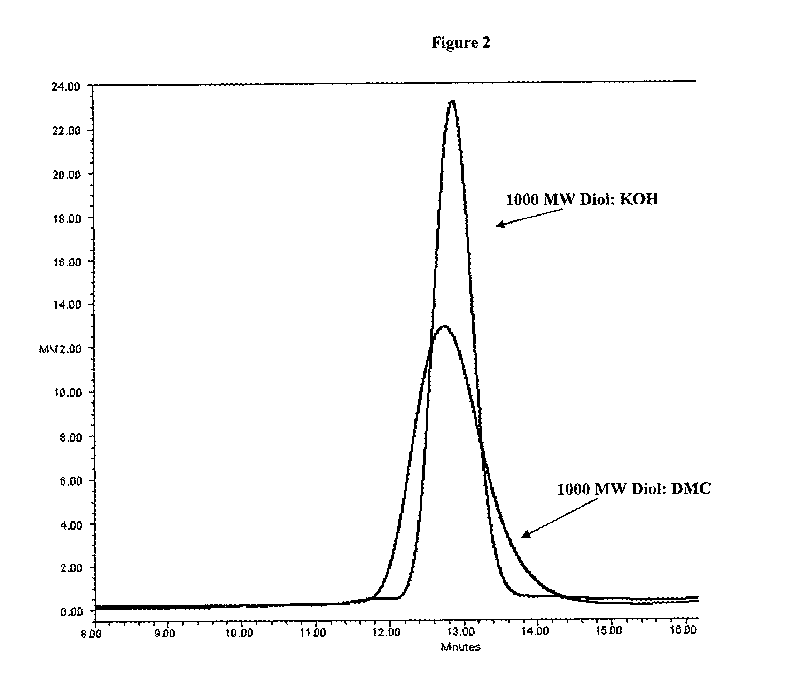

The present inventors have found that the polydispersities of the alkylphenol ethoxylates prepared with a continuous process using DMC catalysis are similar to those obtained from polypropylene oxide polyols and mixed polyethylene oxide-polypropylene oxide polyols. With such polymers, the polydispersities are typically wider for the continuous products (DMC) process than the polydispersities of the products produced in the semi-batch (KOH) process. Figure 1 compares the polydispersity of a 3,200 MW KOH-catalyzed triol with an ethylene oxide content of 11.5 percent and a 3,000 MW DMC-catalyzed triol with an ethylene oxide content of 7.4 percent. Figure 2 compares the polydispersity of 1,000 MW all propylene oxide diols made by KOH catalysis and by DMC catalysis. In both cases, the DMC-catalyzed materials show a broader polydispersity.

As those skilled in the art are aware in transitioning from one manufacturing process to another, one of the key requirements is that the products have similar properties. It is known in the art that the relative surfactancy of a given weight of ethoxylates is related to the product distribution of the ethoxylates and thus it is desirable to maintain consistent properties. The inventive continuous processes provide the desired product quality combined with increases in productivity.

The inventive continuous processes also eliminate the nonproductive sequences required for the semi-batch process. Once the startup is achieved, the reactor is fully utilized for ethoxylation. The catalyst, starter and ethylene oxide are continuously charged with no heat-up or water removal stages. The addition of the starter at ambient temperature is an advantage as the heat of reaction is used to bring it to process temperature. The catalyst in the reactor has on-going activity and new catalyst is continually activated as the process proceeds. The product flows from the reactor and the last traces of ethylene oxide are eliminated either in the piping system, short pipe reactor or in the product analysis tank. If the system is equipped with analytical instruments such as a near-infrared detector, product variability is low as incremental starter and ethylene oxide weight changes can be made to maintain product quality.

At the end of each of these processes, the product may contain a low level of unreacted ethylene oxide. The oxide level continues to decrease as the product flows from the reactor by pipes to product analysis tanks. An alternative to the use of these lines would be a pipe or plug-flow reactor in which no oxide is added. In addition, any residual oxide would continue to be reduced while the alkylphenol ethoxylate was in the product tank.

Preferred initiators or starters (the terms are used interchangeably herein) in the inventive single-stage and multi-stage processes are alkylphenols with the alkyl group having from 1 to 20 carbon atoms and more preferably from 3 to 13 carbon atoms. The alkyl group of the alkylphenol may have a number of carbon atoms in the processes of the present invention in an amount ranging between any combination of these values, inclusive of the recited values. The alkylphenol may optionally be branched. Particularly preferred initiators are nonylphenols, octylphenols and dodecylphenols.

The processes of the present invention may employ any double metal cyanide ("DMC") catalyst. Double metal cyanide complex catalysts are non-stoichiometric complexes of a low molecular weight organic complexing agent and optionally other complexing agents with a double metal cyanide salt, e.g. zinc hexacyanocobaltate. Suitable DMC catalysts are known to those skilled in the art. Exemplary DMC catalysts include those suitable for preparation of low unsaturation polyoxyalkylene polyether polyols, such as disclosed in U.S. Pat. Nos. 3,427,256; 3,427,334; 3,427,335; 3,829,505; 4,472,560; 4,477,589; and 5,158,922, the entire contents of each of which are incorporated herein by reference. The DMC catalysts more preferred in the process of the present invention are those capable of preparing "ultra-low" unsaturation polyether polyols. Such catalysts are disclosed in U.S. Pat. Nos. 5,470,813 and 5,482,908, 5,545,601, 6,689,710 and 6,764,978, the entire contents of each of which are incorporated herein by reference. Particularly preferred in the inventive process are those zinc hexacyanocobaltate catalysts prepared by the processes described in U.S. Pat. No. 5,482,908.

The DMC catalyst concentration is chosen so as to ensure good control of the ethoxylation reaction under given reaction conditions. The catalyst concentration is preferably from 5 ppm to 1,000 ppm, more preferably in the range of from 10 ppm to 500 ppm, and most preferably in the range from 20 ppm to 100 ppm, based on the final ethoxylate weight. The ethoxylation in the process of the present invention may occur in the presence of DMC catalyst in an amount ranging between any combination of these values, inclusive of the recited values.

Although the inventors herein believe that the term "establishing ethoxylation conditions" in an oxyalkylation reactor is self-explanatory, such conditions are established when the reactor temperature, ethylene oxide pressure, catalyst level, degree of catalyst activation, presence of oxyalkylatable compounds within the reactor, etc., are such that upon addition of unreacted ethylene oxide to the reactor, ethoxylation takes place. By the term "continuously introducing" with respect to addition of ethoxylation oxide and starter herein is meant truly continuous, or an incremental addition which provides substantially the same results as continuous addition of these components.

The alkylphenol ethoxylates produced by the inventive process preferably have a number average molecular weight of from 150 Da to 20,000 Da, more preferably from 250 Da to 12,000 Da, most preferably from 350 Da to 750 Da. The alkylphenol ethoxylates produced by the inventive process may have a number average molecular weight ranging between any combination of these values, inclusive of the recited values. The alkylphenol ethoxylates produced by the inventive processes may preferably find use in or as surfactants.

The inventive multi-stage processes may take place in a continuous stirred tank reactor (1^{st} stage) connected to a tubular (pipe or flow) reactor (2^{nd} stage) or connected to a second CSTR or other type of reactor. The multi-stage process may also take place in a partitioned CSTR reactor allowing a single vessel to serve as a multi-stage CSTR by partitioning the reactor into separate compartments.

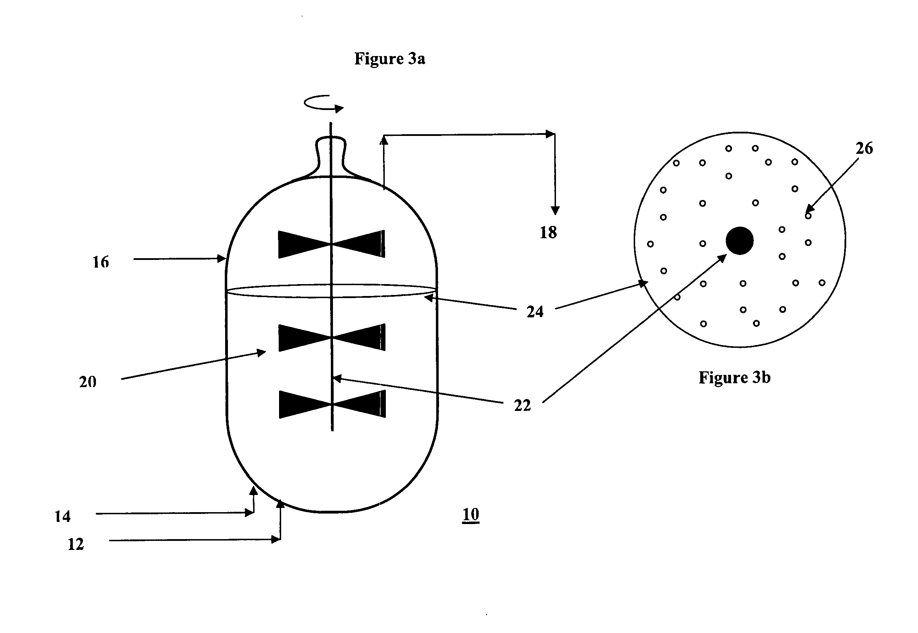

Figure 3a is a diagram of such a partitioned CSTR reactor **10** useful in the present invention. The CSTR reactor is physically partitioned into two (or more) compartments by a perforated plate **24** (or plates) shown in Fig. 3b. Catalyst/initiator slurry **12** and ethylene oxide **14** are fed into the lower compartment and reacted in that chamber to form the backbone of the polyol molecule. This polyol intermediate is forced to flow upward through the openings **26** of the perforated plate **24** to the upper compartment continuously. A separate feed **16** can be introduced into the upper compartment for a second reaction. The final product **18** is overflowed from the top of the reactor **10**. Additional stages may be added similarly, if required. The agitator blades **20** for both compartments are anchored in a common shaft **22**. Not shown are re-circulation loops (for cooling) and/or heat exchange surfaces (cooling jacket or coils) which may be added as needed.

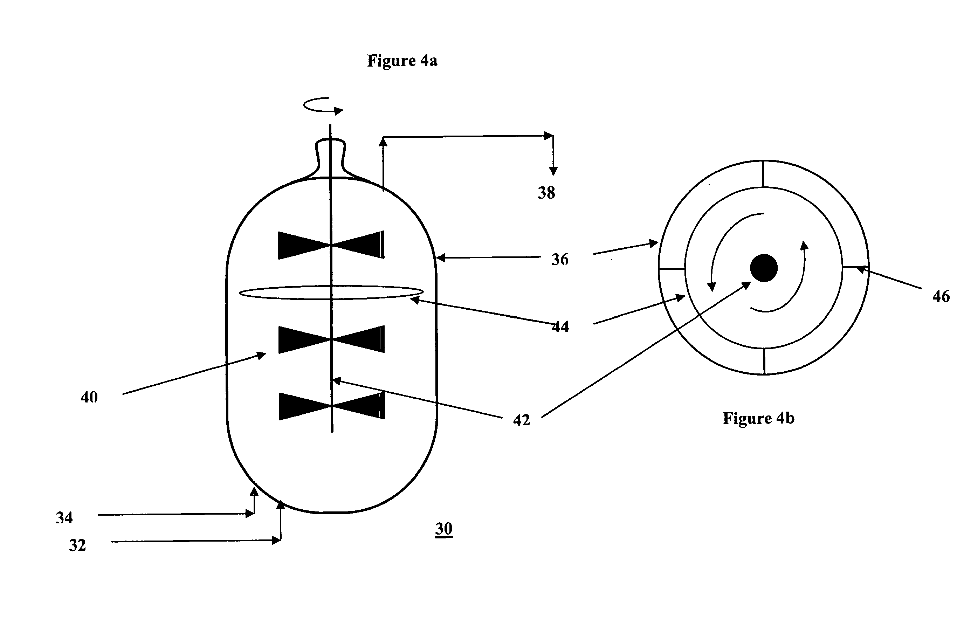

Alternatively, as shown in Figure 4a, a rotation disk **44** (or disks) may be anchored on the agitator shaft **42** and serve as a partition (or partitions) for the reactor **30**. As described above, catalyst/initiator slurry **32** and ethylene oxide **34** are fed into the lower compartment and reacted in that chamber to form the backbone of the polyol molecule. As shown in Figure 4b, the gap between the reactor wall **36** and the rim of disk **44** serves as an open space for liquid flow from the lower to the upper compartment. This design is more flexible because the location of the disk **44** (i.e. the volume ratio of the partitioned compartments) can be adjusted. A separate feed can be introduced into the upper compartment for a second reaction. The final product **38** is overflowed from the top of the reactor **30**. Baffles 46 may optionally be added.

It is expected that one skilled in the art could combine different types of reactors to produce a multi-stage reactor train which would give the desired product distribution. For example, other type of reactors that were originally developed for the semi-batch alkoxylation processes could be easily modified for the continuous process including Venturi Loop reactors and spray tower loop reactors as given in: http://adt.lib.swin.edu.au/uploads/approved/adt-VSWT20050610.140607/public/ 02chapterl-4.pdf. Staged reactors and the compartmentalized reactors, such as those disclosed in U.S. Pat. No. 7,012,164, (See Figures 1-4) and variants thereof should be especially suitable for use in the inventive processes for producing ethoxylates.

### EXAMPLES

The present invention is further illustrated, but is not to be limited, by the following examples. All quantities given in "parts" and "percents" are understood to be by weight, unless otherwise indicated. In all examples herein using a DMC catalyst, the catalyst was made according to U.S. Pat. No. 5,482,908.

### Examples C1, 2 and 3

Example C1 was a product from a commercial process utilizing KOH/NaOH as catalyst and having a polydispersity of 1.08.

In Example 2, a 9.5 mole nonylphenol ethoxylate was produced by a two-stage process as follows: a 9.5 mole nonylphenol ethoxylate was charged to a one-gallon CSTR stainless steel reactor equipped with a mechanical agitator and to a two-gallon CSTR stainless steel reactor equipped with a mechanical agitator. Both reactors were slowly heated. After the reactor temperature reached 130°C, an initial charge of ethylene oxide was charged to the reactor over several minutes. After 10 minutes, the pressure in the reactor decreased indicating that the DMC catalyst was active. The ethylene oxide feed was restarted and set at a rate of 14.4 g/min (equivalent to a two hour residence time). After establishing the oxide feed, a feed containing nonylphenol and 133 ppm DMC catalyst was started at a rate of 15.2 g/min.

The DMC catalyst was added to the nonylphenol as a dry powder and remained dispersed in the nonylphenol by constant agitation of the nonylphenol /DMC catalyst feed vessel.

When the pressure in the reactor reached 50 psia, a valve at the top of the reactor was opened to a back pressure regulator and the contents of the liquid full reactor were allowed to flow out of the reactor. The polyether coming out of the one-gallon reactor was directed into bottom of the two-gallon reactor. The ethylene oxide feed (14.4 g/min) to the two-gallon reactor was started, equivalent to a 2.7 hour residence time in the two-gallon reactor. The polyether was continuously removed from the two-gallon reactor by opening a valve at the top of the reactor to a back pressure regulator and allowing the contents of the liquid full reactor to flow out of the reactor. The polyether coming out of the reactor was passed through a steam-heated line before being collected in a heated and stirred jacketed vessel. The feeds were continued for 9 hours at which point the feeds to both reactors were stopped. A sample of the collected product had a polydispersity of 1.15.

In Example 3, a 9.5 mole nonylphenol ethoxylate was produced by a two stage process as follows: using similar conditions as in Example 2, a mixture of nonylphenol and DMC catalyst that contained 133 ppm catalyst was charged to the catalyst feed vessel. The ethylene oxide (11.5 g/min) and nonylphenol/DMC catalyst mixture (15.1 g/min) feeds were started to the one-gallon reactor, equivalent to a residence time of 2.2 hours in the one-gallon reactor. A short time later, the ethylene oxide feed (17.2 g/min) to the two-gallon reactor was started, equivalent to a 2.7 hour residence time in the two-gallon reactor. The polyether was continuously removed from the two-gallon reactor and collected in a manner similar to Example 2. The feeds were continued for 8 hours at which point the feeds to both reactors were stopped. A sample of the collected product had a polydispersity of 1.14.

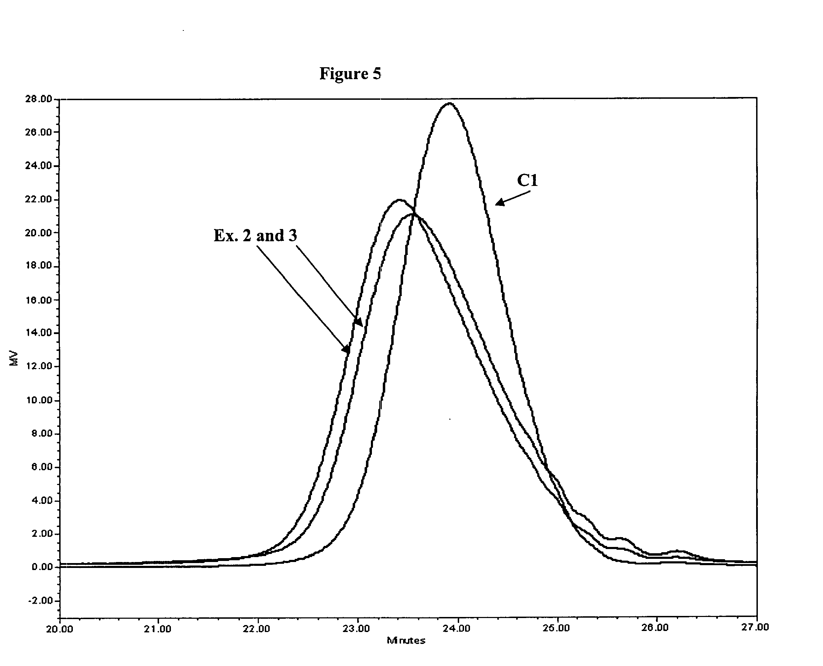

Figure 5 compares the polydispersity of polyols produced by base catalysis and the inventive multi-stage DMC-catalyzed process. As can be appreciated by reference to Figure 5, the polydispersities of the products made by the inventive multi-stage process are slightly broader than that of the product made by the base-catalyzed process.

The foregoing examples of the present invention are offered for the purpose of illustration and not limitation. It will be apparent to those skilled in the art that the embodiments described herein may be modified or revised in various ways without departing from the spirit and scope of the invention. The scope of the invention is to be measured by the appended claims.

## Claims

1. A multi-stage continuous process for the production of an alkylphenol ethoxylate comprising:
preparing a mixture of an alkylphenol and a double metal cyanide ("DMC") catalyst; establishing ethoxylation conditions in a first continuous stirred tank reactor ("CSTR");
continuously feeding ethylene oxide and the mixture of alkylphenol and DMC catalyst to the first CSTR reactor under conditions suitable to produce an ethoxylate;
continuously feeding the reaction mixture from the first CSTR reactor and further ethylene oxide to a second CSTR reactor or to a tubular reactor under conditions suitable to produce an alkylphenol ethoxylate; and
continuously withdrawing the alkylphenol ethoxylate from the second CSTR reactor or tubular reactor to a collection vessel.

2. The multi-stage continuous process according to Claim 1, wherein the alkylphenol alkyl group contains from 1 to 20 carbon atoms.

3. The multi-stage continuous process according to Claim 1, wherein the alkylphenol alkyl group contains from 3 to 13 carbon atoms.

4. The multi-stage continuous process according to Claim 1, wherein the alkylphenol is selected from nonylphenols, octylphenols and dodecylphenols.

5. The multi-stage continuous process according to Claim 1, wherein the tubular reactor is a pipe reactor or a plug flow reactor.

6. The multi-stage continuous process according to Claim 1 further including a step of continuously feeding the ethoxylate from the second CSTR reactor or tubular reactor to a third reactor under conditions suitable to produce an alkylphenol ethoxylate.

7. The multi-stage continuous process according to Claim 6, wherein the third reactor is a CSTR reactor or a tubular reactor.

8. The multi-stage continuous process according to Claim 1, wherein the alkylphenol ethoxylate has a number average molecular weight of from about 150 Da to about 20,000 Da.

9. The multi-stage continuous process according to Claim 1, wherein the alkylphenol ethoxylate has a number average molecular weight of from about 250 Da to about 12,000 Da.

10. The multi-stage continuous process according to Claim 1, wherein the alkylphenol ethoxylate has a number average molecular weight of from about 350 Da to about 750 Da.

11. A multi-stage continuous process for the production of an ethoxylate comprising:
preparing a mixture of an alkylphenol and a double metal cyanide ("DMC") catalyst; establishing ethoxylation conditions in a first portion of a partitioned continuous stirred tank reactor ("CSTR");
continuously feeding ethylene oxide and the mixture of alkylphenol and DMC catalyst to the first portion of the CSTR reactor under conditions suitable to produce an ethoxylate;
continuously forcing the reaction mixture from the first portion of the CSTR reactor into a second portion of the CSTR reactor and adding further ethylene oxide under conditions suitable to produce an alkylphenol ethoxylate; and
continuously withdrawing the alkylphenol ethoxylate from the second portion of the CSTR reactor to a collection vessel.

12. The multi-stage continuous process according to Claim 11, wherein the continuous stirred tank reactor ("CSTR") is partitioned with one or more perforated plates and/or rotation disks.

13. The multi-stage continuous process according to Claim 11, wherein the alkylphenol alkyl group contains from 1 to 20 carbon atoms.

14. The multi-stage continuous process according to Claim 11, wherein the alkylphenol alkyl group contains from 3 to 13 carbon atoms.

15. The multi-stage continuous process according to Claim 11, wherein the alkylphenol is selected from nonylphenols, octylphenols and dodecylphenols.

16. The multi-stage continuous process according to Claim 11, wherein the alkylphenol ethoxylate has a number average molecular weigh of from about 150 Da to about 20,000 Da.

17. The multi-stage continuous process according to Claim 11, wherein the alkylphenol ethoxylate has a number average molecular weigh of from about 250 Da to about 12,000 Da.

18. The multi-stage continuous process according to Claim 11, wherein the alkylphenol ethoxylate has a number average molecular weigh of from about 350 Da to about 750 Da.

19. A single stage continuous process for the production of an alkylphenol ethoxylate comprising:
preparing a mixture of an alkylphenol and a double metal cyanide ("DMC") catalyst; establishing ethoxylation conditions in a reactor;
continuously feeding ethylene oxide and the mixture of alkylphenol and DMC
catalyst to the reactor under conditions suitable to produce an ethoxylate; continuously feeding further ethylene oxide to the reactor under conditions suitable to
produce an alkylphenol ethoxylate; and continuously withdrawing the alkylphenol ethoxylate from the reactor to a collection vessel.

20. The multi-stage continuous process according to Claim 19, wherein the alkylphenol alkyl group contains from 1 to 20 carbon atoms.

21. The multi-stage continuous process according to Claim 19, wherein the alkylphenol alkyl group contains from 3 to 13 carbon atoms.

22. The single-stage continuous process according to Claim 19, wherein the alkylphenol is selected from nonylphenols, octylphenols and dodecylphenols.

23. The single-stage continuous process according to Claim 19, wherein the alkylphenol ethoxylate has a number average molecular weight of from about 150 Da to about 20,000 Da.

24. The single-stage continuous process according to Claim 19, wherein the alkylphenol ethoxylate has a number average molecular weight of from about 250 Da to about 12,000 Da.

25. The single-stage continuous process according to Claim 19, wherein the alkylphenol ethoxylate has a number average molecular weight of from about 350 Da to about 750 Da.

26. The single-stage continuous process according to Claim 19, wherein the reactor is a pipe reactor or a plug flow reactor.

27. In a process for the production of a surfactant, the improvement comprising including the alkylphenol ethoxylate produced by the multi-stage continuous process according to Claim 1.

28. In a process for the production of a surfactant, the improvement comprising including the alkylphenol ethoxylate produced by the multi-stage continuous process according to Claim 11.

29. In a process for the production of a surfactant, the improvement comprising including the alkylphenol ethoxylate produced by the single-stage process continuous process according to Claim 19.
